# EUROPEAN PATENT SPECIFICATION

(11) **EP 3 003 460 B1**
(45) Date of publication and mention of the grant of the patent: **03.01.2018**
(21) Application number: 14736971.4
(22) Date of filing: 27.05.2014
(51) Int. Cl.: A61M 39/10, A61M 39/18, F16L 29/00

(54) **CONNECTING DEVICE**
VERBINDUNGSANORDNUNG
DISPOSITIF DE CONNEXION

(30) Priority: 28.05.2013 IT RM20130308
(43) Date of publication of application: 13.04.2016
(73) Proprietor: Glomeria Therapeutics S.r.l., 66100 Chieti (IT)
(72) Inventor: ARRIZZA, Fabio, I-66100 Chieti (IT); ARDUINI, Arduino, I-66100 Chieti (IT)
(74) Representative: Leone, Mario
(86) International application number: PCT/IB2014/061750
(87) International publication number: WO 2014/191916

(56) References cited:
- JP-A- S5 397 293
- US-A- 4 022 205
- US-A- 5 492 147
- US-A1- 2003 060 804

## Description

The present invention has as subject a connecting device, in particular for connecting therebetween the two ends of respective ducts with tubular shape.

By way of example and not with limitative purpose, the ducts which can be connected therebetween preferably are two flexible ducts, for example made of plastic or composite material; they are joined on the respective tubular ends so as to allow the circulation of any fluid from a duct to the other one through the so-implemented connection, without such fluid can be polluted by possible impurities lying near said tubular ends which, before the connection thereof, must be sealingly closed and be fluid-tight.

It is meant that, generally, the fluid could be both a liquid and a gas.

A preferred version of such connecting device implements the hydraulic communication between two catheters, for example with the purpose of performing, through said catheters, the insertion and removal of fluids from a living body.

A particular application example is constituted by the connection between the end of a catherer branching from a bag containing a physiological solution with possible drugs, or plasma, blood or other fluids, and the end of another catherer destined to transport such liquid in a living body, to perform any type of treatment

An application provides the use of the present connecting device in the field of dialysis, in particular the peritoneal dialysis, or in other several uses in medical field.

The known devices do not prevent with sufficient certainty the contact between the fluids or the atmosphere and the inner space to the tubular ends to be connected, or they result to be difficult to be used by the user.

By referring to the peritoneal dialysis or other clinical treatments, in the first case the contamination of the fluid by outer, even pathogen agents could occur, which would involve a dangerous peritonitis, whereas in the second case the user, often with limited cognitive and motory capabilities, can perform use errors and jeopardize the dialytic treatment itself. A complicated use can also prevent from adopting such treatment on said subjects.

The same risks can be connected to the administration of blood or drugs or to the procedures of parenteral nutrition.

The problem to keep pure a fluid through the connection of two ducts is however felt in other fields requesting a very precise control of the composition of such fluid, in order to not compromise organic, chemical, physical processes or others, for example in the food field, wherein a device is requested guaranteeing the maximum safety in a single-use item.

The US patent Nr. 4,022,205 and the Japanese patent Nr. JP S53 97293 A describe a connector for two tubular ducts, respectively.

The technical problem underlying the present invention is to provide a connecting device allowing to obviate the drawbacks mentioned with reference to the known art.

Such problem is solved by a connecting device to connect therebetween the respective ends of two ducts, as defined by the enclosed claim 1. The main advantage of the device according to the present invention lies in the fact of guaranteeing the requested connection without the inside portion of the ducts comes in contact with the atmosphere and with the polluting agents before the complete connection thereof, by preventing possible contaminations by pathogen, or however extraneous agents and by keeping the connection operation simple to be performed.

Furthermore, in relation to the application in the peritoneal dialysis, the present invention reduces considerably the constructive complexity of the whole device which then can be wholly single-use.

The present invention will be described hereinafter according to a preferred embodiment thereof, provided by way of example and not with limitative purposes with reference to the enclosed drawings wherein:

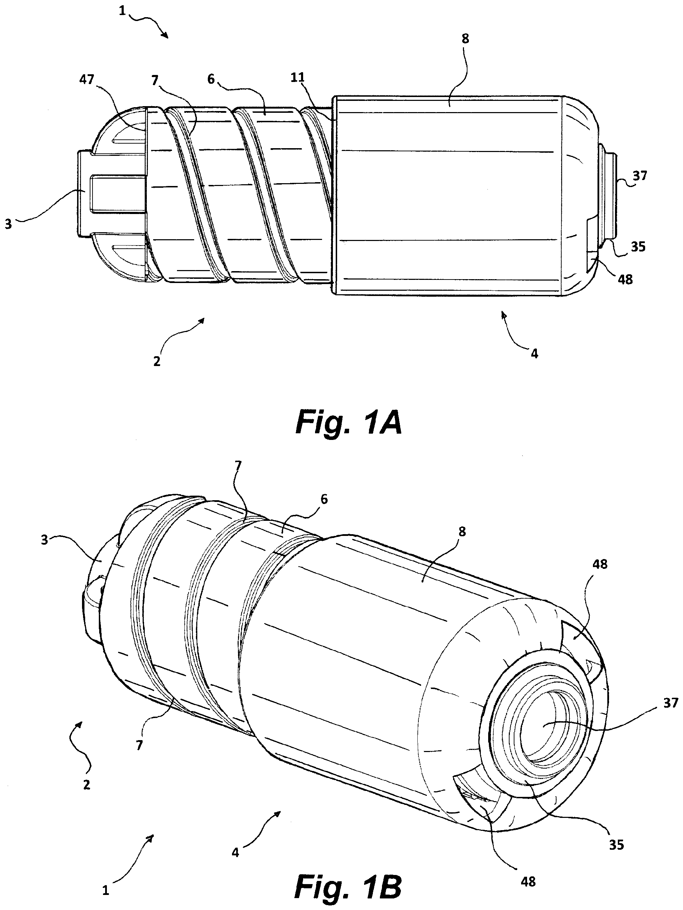

- figures 1A, 1B and 1C show an embodiment example of connecting device according to the invention respectively with a side view, an axonometry from right and an axonometry from left, the device being in a configuration preliminary to the connection;
- figures 2A, 2B and 2C show side sections taken according to a longitudinal axial plane of the connecting device as shown in figures 1A, 1B and 1C respectively, in a configuration preceding the connection;

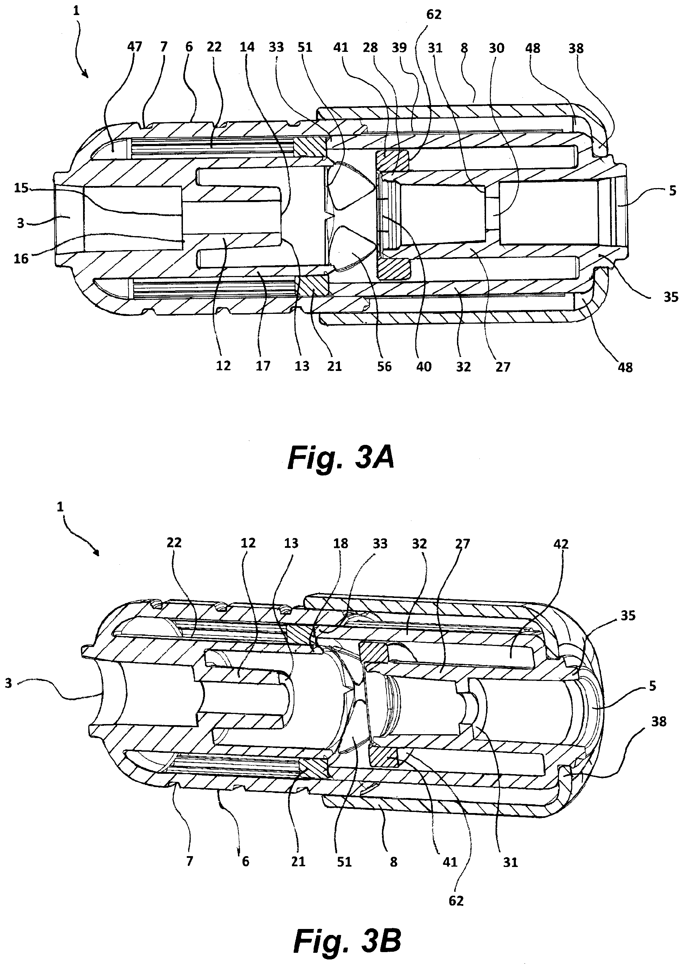

- figures 3A and 3B show side sections taken according to a longitudinal axial plane of the connecting device, in a side and axonometric view from left respectively, to illustrate a first connecting phase;
- figures 4A and 4B show a side section taken according to a longitudinal axial plane of the device of the previous figures respectively, in a side and axonometric view from left, to illustrate a second connecting phase;
- figures 5A, 5B, 5C and 5D show side sections taken according to different axial longitudinal planes of the connecting device, in a side view and in three different axonometries, with the device in an intermediate configuration;
- figures 6A and 6b show side sections taken according to a longitudinal axial plane of the connecting device as shown in figures 1A and 1B respectively, in a configuration more advanced towards the connection;
- figures 7A and 7B show side sections taken according to a longitudinal axial plane of the connecting device, in a side and axonometric view from left respectively, to illustrate a completed connection phase;
- figures 8A, 8B and 8c show a side section taken according to a longitudinal axial plane, a front view from right and an axonometry from right, with respect to the previous figure, of a first connecting member of the device of the previous figures;
- figures 9A, 9B and 9C show a side section taken according to a longitudinal axial plane, a side view and an axonometry of an inner portion of a second connecting member of the device of the previous figures;
- figures 10A and 10B show respectively an axonometry and a side section taken according to a longitudinal axial plane of an outer portion of a second connecting member of the device of the previous figures;
- figures 11A and 11B show respectively the axonometries of a component of the herein described connecting and of a portion thereof; and

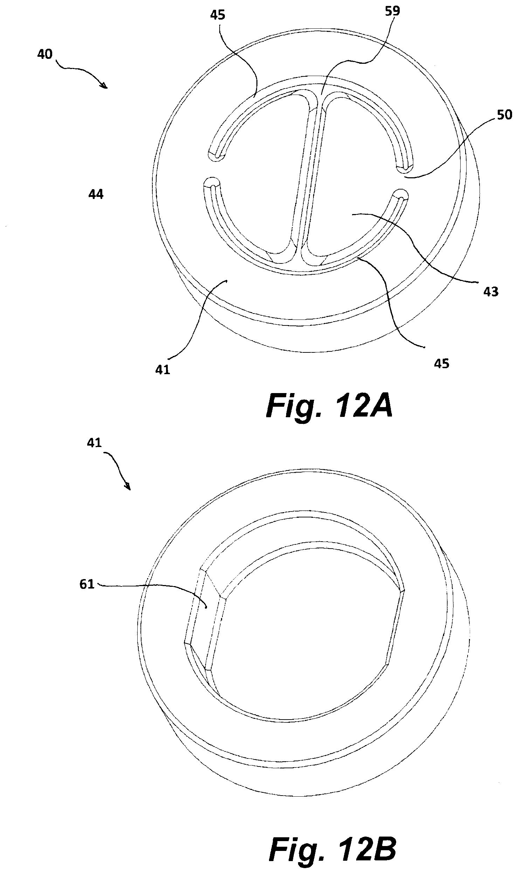

- figures 12A and 12B show respectively the axonometries of another component of the herein described connecting device and of a portion thereof.

By referring to the figures, a connecting device is designated as a whole with 1. It is arranged to implement a stable connection between the respective ends of two distinct flexible ducts, with tubular ends to be joined therebetween, with the purpose of allowing the passage of a fluid through the connection thereof, such as a gas, a liquid, a solution for medical purposes, an organic fluid, without the fluid comes in contact with outer agents.

To this purpose the connecting device 1 comprises a first connecting member 2 which in turn comprises a first connecting section 3 represented by a tubular recess apt to receive a first not represented duct end.

Analogously, the device 1 comprises a second connecting member 4 which in turn comprises a first connecting section 5 represented by a tubular recess apt to receive a not represented duct end.

The two connecting sections 3, 5 are formed so as to adapt to the tubular end of the destination duct by mechanical interference and air-or water-tight, that is tight to any liquid; in principle they can have the same diameter and the same depth if they have to be coupled to ducts equal therebetween, but they can also have different sizes and provide different fastening means.

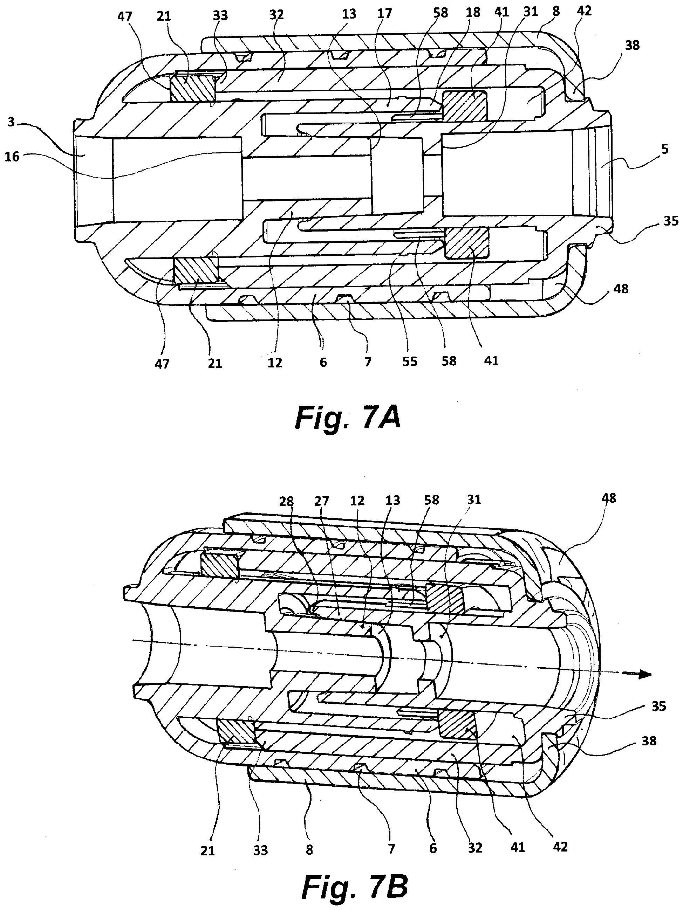

In the present embodiment, each connecting device 2, 4 has substantially a cylindrical symmetry around a longitudinal axis A (represented in figure 7B), representing the connection axis between the ducts, even if the latter could be misaligned therebetween.

The connecting devices 2, 4 are apt to cooperate mechanically therebetween in the axial sliding, penetrating one into the other one as it will appear with greater clarity from the following description.

A preferred way to implement such sliding consists in equipping a connection member with a sliding guide, for example a groove formed on the outer surface of an outer cylindrical casing of a connecting member, apt to cooperate with a corresponding rib or with one or more teeth implemented at the inner surface of the outer cylindrical casing of the other connecting member.

Such guide can even be helical, thus producing a screw coupling between the connecting devices, even involving an axial rotation in addition to said sliding.

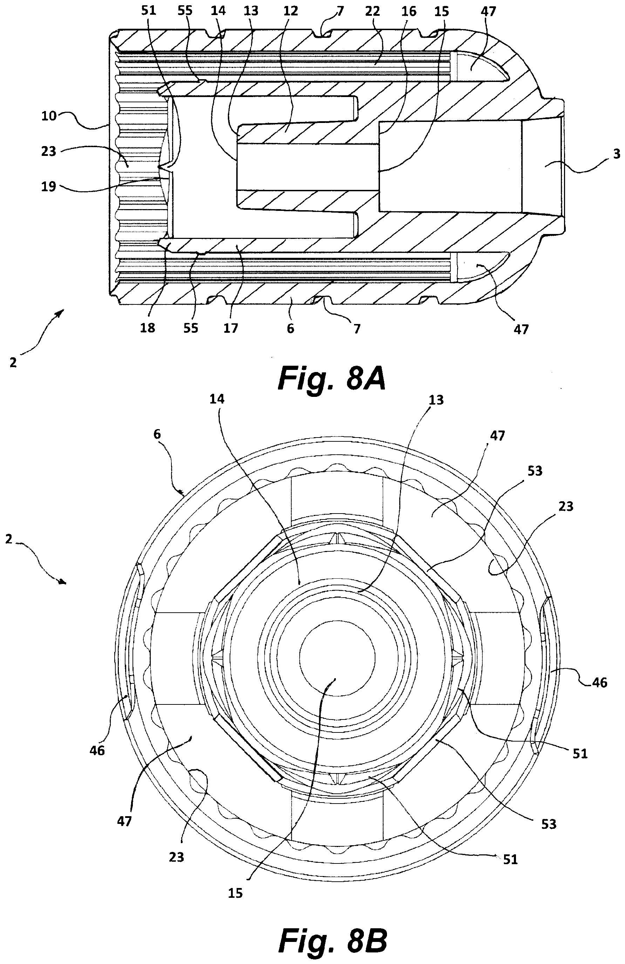

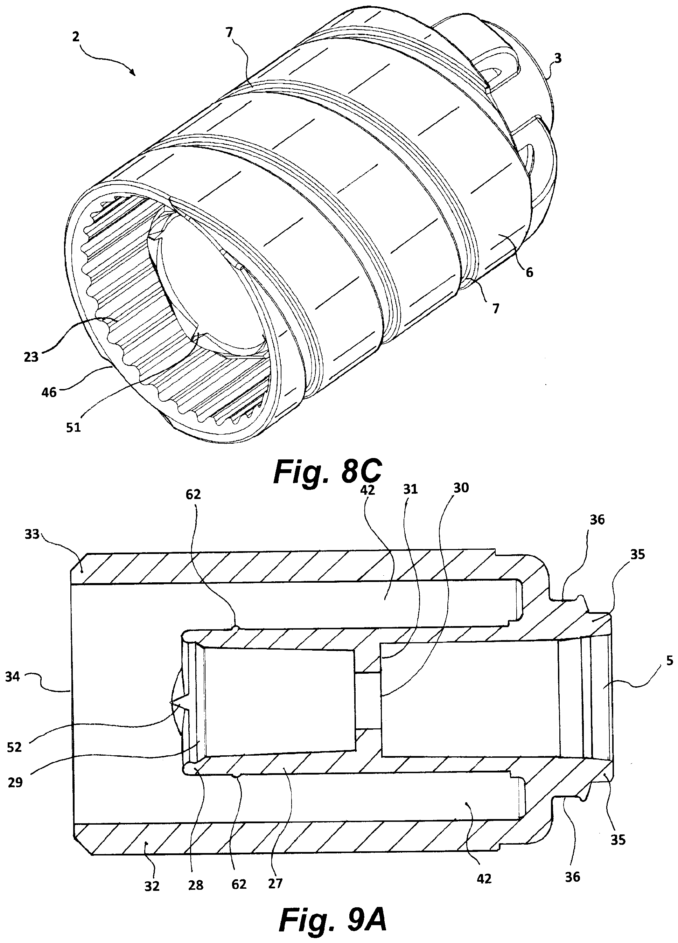

In the present embodiment, the first connecting device 2 (figure 8A and 8B) has a first outer cylindrical casing 6 comprising, on its own outer surface, a pair of helical grooves 7 working with double principle with mouths 46 on symmetrically opposed sides.

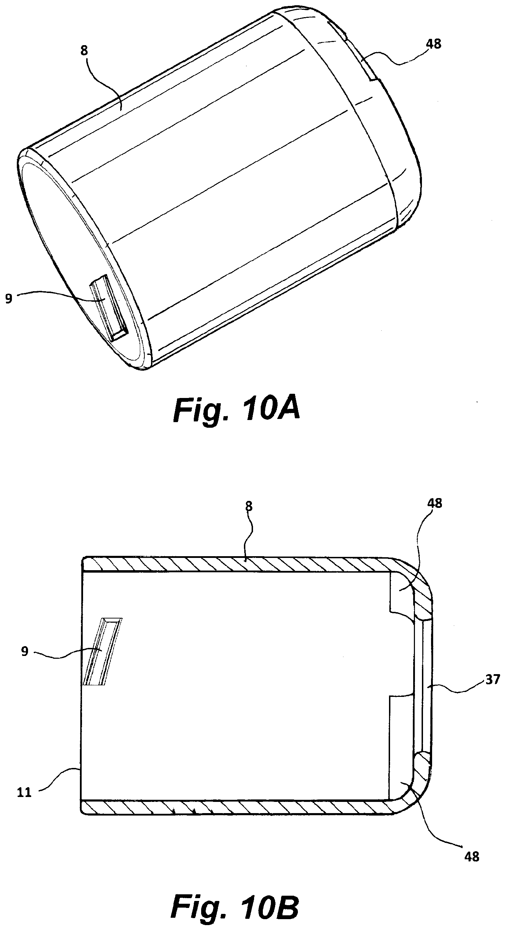

On the contrary, the second connecting device 4 (figure 10A and 10B) has a second outer cylindrical casing 8 comprising, on its own inner surface near the ending edge, a pair of opposed connecting teeth 9, apt to cooperate with said grooves 7 to implement a screw coupling as previously explained.

The ends of the connecting devices 2, 4 opposite to the respective connecting sections 3, 5, and respectively designated with 10 and 11, are open and they allow inserting the first connecting member 2 inside the second one, to implement said screw coupling.

The first connecting device 2 then comprises
a first tubular terminal 12 formed therein, equipped with a first ending circular edge 13 with a prefixed diameter defining a first connecting opening 14 faced towards the open end 10. The first circular edge 13 lies inside the connecting member 2 in a moved-back position with respect to said open end or mouth 10.

The first tubular terminal 12 is in axis with the first outer casing 6 which then includes it wholly and it is in hydraulic communication with the respective first connecting section 3 through a first passage hole 15 formed in a first intermediate wall 16 constituting the bottom of the first section 3. The first terminal 12 and the corresponding first section 3, in this embodiment example, are coaxial.

The first connecting device 2 further comprises a first tubular casing 17 concentric to said first tubular terminal 12 and to the first outer casing, being arranged in intermediate position therebetween (figure 8A), equipped with a third ending edge 18 defining an additional or third connecting opening 19, opposing to a second connecting opening of the second connecting member 4, which will be detailed shortly.

Said third opening 19 is positioned inside the outer casing 6, in an intermediate position between the mouth 10, with respect thereto it is moved back, and said first connecting opening 14.

The third edge 18 has not a perfectly circular contour ha, but thereon some members of the connecting device 1 are formed.

In particular, along said contour four first sharp profiles 51 and four first plane connecting surfaces 53, alternated (figure 8B), are provided; the first sharp profiles 51 have a shape like a cusped blade with the top point extending in the longitudinal direction defined by the first respective tubular casing 17; the function thereof will be described hereinafter.

The first four connection surfaces 53 instead have the purpose of transforming the first tubular casing 17 in a member of prismatic pair, for the reason which will be explained hereinafter.

On the outer surface of the first tubular casing 17, which is smooth, a first toroidal tooth 55 is further formed, near but however at a certain distance from the rim of the first edge 18, to avoid that the first ring 21 goes back and therefore that the barrier 21 breaks, in an assembling phase or due to an unintentional action, not determined by the operation of the device 1.

The first ring 21 is fitted with a certain interference on the seat thereof, to avoid a detachment of the first barrier 20.

The inner surface of the first outer casing 6 has an axial ruling, formed by a plurality of rectilinear grooves 23.

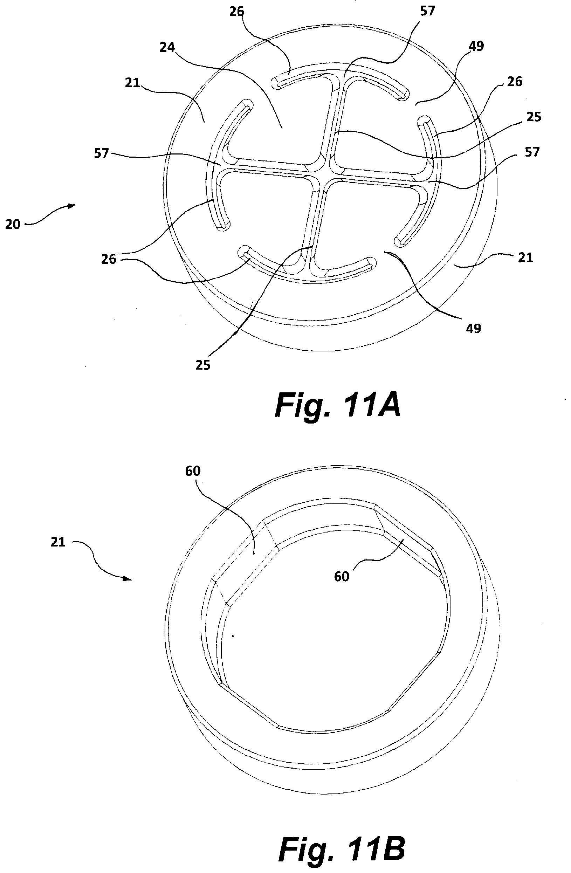

The third connecting opening 19 is sealed by a first breakable barrier 20 (figure 11A) connected to said third circular ending edge 18, by preventing the duct connected to the first connecting section 3 from being in communication with outside through the first connecting terminal 12, remaining protected by said first barrier 20.

This first breakable barrier 20, in this embodiment example, comprises a first ring 21 which is arranged outwardly the first tubular casing 17 and it is arranged to slide in a first air gap 22 which is formed between the first tubular casing 17 and the first outer casing 6.

The bottom of this first air gap 22, at the first connecting section 3, is provided with first discharging openings 47, to prevent the formation of an air bag able to obstruct the feeding of the first ring 21.

It is to be noted that at first the first ring 21 is fitted on the first tubular casing 17 at the edge thereof 18, between the rim of this one and the first toroidal tooth 55 which keeps it in position during the assembly, to prevent involuntary breaks of the first barrier 20.

The first ring 21, in turn, has a predetermined thickness to adapt to the position of the toroidal tooth 55, and a not circular contour, but which has at regular intervals second plane connecting surfaces 60, aimed at coupling in a prismatic relation with said first connecting surfaces 53 thereto they are wholly complementary, this prevents the first ring 21 and the first breakable barrier 20 generally from rotating with the first tubular casing 17 as pin.

The breakable barrier 20 further comprises a first membrane 24 extending over the whole area defined by said ring 21, so as to obstruct completely the third connecting opening 19. On the first membrane 24 first rupture lines 25, diametrally transversal to the membrane 24 with a cross-like configuration, will cause the opening of the barrier 20, and second peripheral rupture lines 26, arranged on the circular periphery of the membrane 24, to ease the detachment between this one and the first ring 21.

These two types of rupture lines are destined to determine a localized separation between the first membrane 24 and the first ring 21 and between portions of the same first membrane 24.

In order to keep such portions of the first membrane 24, which will open like a petal, joined to the ring, first anchoring means 49 is provided, determined by the interruptions of said first peripheral rupture lines 26.

The separation of said membrane portions, hereinafter first petal-like strips 56, starts at first points 57 for triggering the separation, which in the present example are determined by the intersections between the first cross rupture lines 25 and the first peripheral rupture lines 26.

It is to be noted that the position of said first triggering points will correspond exactly to that of the points of the first sharp profiles 51 on the third edge 18 of the first tubular casing 17. For this reason, that is to keep this correspondence, the ring 21 has to be prevented from rotating. Therefore, the cooperation between the first and the second connecting surfaces 53, 60 allows performing automatically the assembly of the first breakable barrier 20 in a correct position.

The rupture mechanism of the first breakable barrier 20 is the following one: when the ring 21 is pushed into sliding towards the first connecting section 3 from the axial sliding between the connecting devices 2, 4, the first rupture lines 25 and 26, due to the effect of the incisions caused by the first sharp profiles 51 on the first triggering points 57 of the first membrane 24, separate, but the membrane 24, separated in four petal-like strips 56, is dragged too by the ring 21 leaving uncovered the third opening 19 which before was sealed.

The second connecting device 4 (figure 9A) comprises instead a second tubular terminal 27 formed therein, equipped with a second ending edge 28 with prefixed diameter.

To this purpose, the second connecting opening 29 is directed towards the respective open end 11 of the second connecting member 4, and the second edge 28 lies inside the second connecting member 4, in moved-back position with respect to the respective second open end or mouth 11.

The second tubular terminal 27 is in axis with the second outer casing 8, which then includes it entirely, and it is in hydraulic communication with the respective second connecting section 5 through a second passage 30 formed in a second intermediate wall 31 constituting the bottom of the second section 5.

The second terminal 27 and the second section 5, in this embodiment example, are coaxial therebetween and even with all the cylindrical/tubular components of the first connecting member 2 with respect to the longitudinal axis A (figure 7A).

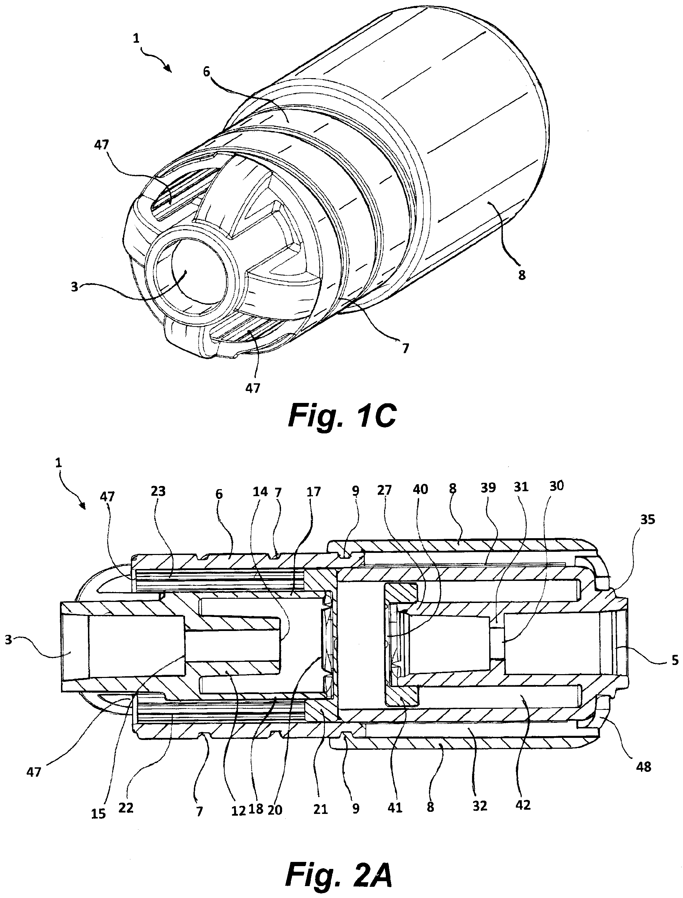

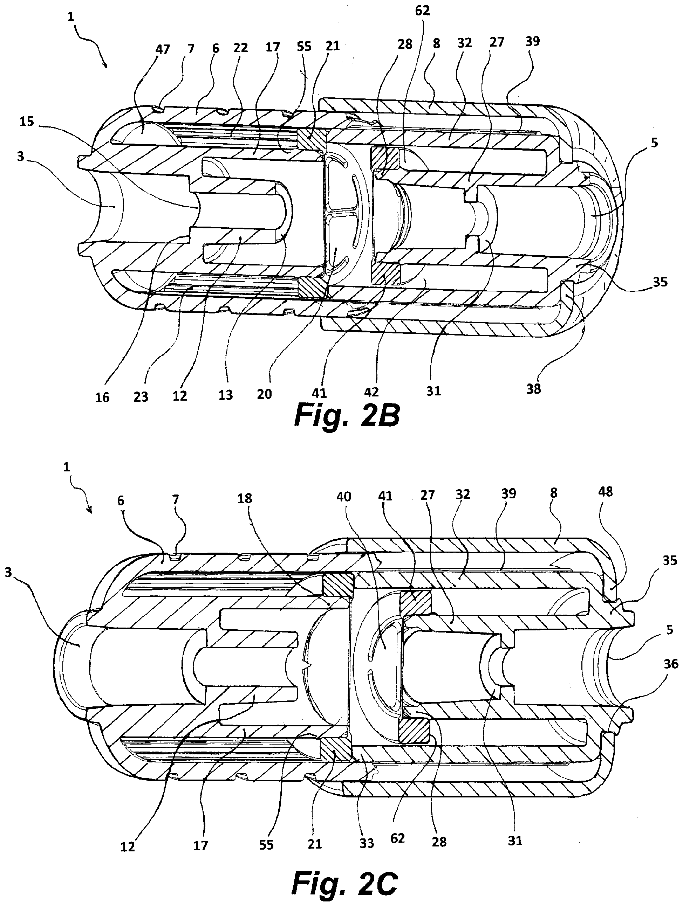

The second connecting device 4 further comprises a second tubular casing 32 (figure 2A) concentric to said first tubular terminal 12, equipped with a fourth circular ending edge 33 di defining a fourth connecting opening 34 destined to be inserted in the first mouth 10 of the first connecting member 2.

The fourth opening 34 is positioned slightly inside the second outer casing 8, in an intermediate position between the second mouth 11, with respect thereto it is only slightly moved back, and said second connecting opening 29.

Differently from the first connecting member 2, which advantageously can be constructed in one single piece with the exception of the barrier 20, the second connecting device 4 of the present embodiment is constituted at least by two distinct members plus the respective barrier.

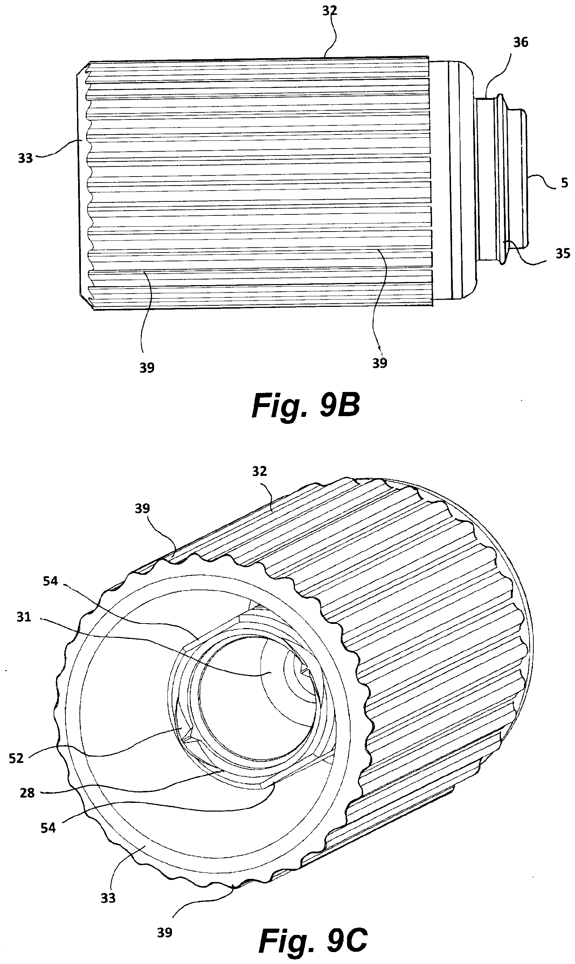

In fact, the second outer casing 8 is separated from the rest, and it is free to rotate around said second tubular casing 32. The two components are represented separately in figures 9a, 9b (body of the second connecting member 4) and 10A, 10B (outer casing 8).

To this purpose, the second connecting device 4, at the second connecting section 5, comprises a projecting and cylindrical spigot 35 enclosing the inlet of the connecting section 5.

A sliding seat 36, implemented by an annular groove, is formed on the outer surface of the spigot 35.

Equally, the second outer casing, opposite to the second mouth 11, has an assembly opening 37, centred axially and equipped with a sliding edge 38 apt to be inserted, thanks to the elastic joint by exploiting the elasticity of the material thereof the outer casing 8 is made, inside the sliding seat 36.

This allows the second outer casing 8 to rotate with respect to the body of the second connecting member 4 to act as fastening bushing; in this way, as explained in additional details hereinafter, the body of the connecting member 4 can move only axially during the screw coupling between the outer casings 6, 8 of the connecting devices 2, 4, whereas the second outer casing 8 is free to rotate, for example as rotated manually by a user.

The outer surface of the second tubular casing 32, in turn, is ruled due to the presence of a plurality of rectilinear ribs 39 apt to cooperate with said rectilinear grooves 23 formed on the inner surface of the first outer casing 6, thus determining a prismatic pair preventing the body of the second connecting member 4 from rotating during the screw coupling.

As the third edge 18, neither the second edge 28 has a perfectly circular contour, but thereon some members of the connecting device 1 are formed.

In particular, along said contour, two second sharp profiles 52 are provided, separated by two third plane connecting surfaces 54 (figure 9C), symmetric therebetween; the second sharp profiles 52 have a shape like a cusped blade with the top point extending in the longitudinal direction defined by the second respective tubular terminal 27; the function thereof will be explained hereinafter even if it is analogous to that described with reference to the first sharp profiles 51.

Equally, the second connecting surfaces 53 instead have the aim of transforming the second tubular terminal 27 in a member of prismatic pair, for the reason which will be explained hereinafter.

In the second connecting member 4, the second connecting opening 29 is sealed by a second breakable barrier 40 connected to said circular ending edge 28, by preventing the duct connected to the second connecting section 5 from being in communication with outside through the second connecting terminal 27, which remains protected by this barrier 40.

The second breakable barrier 40, in this embodiment example, comprises a second ring 41 which is arranged outside the second connecting terminal 27 and it is arranged to slide in a second air gap 42 which is formed between the second tubular casing 32 inside and the second connecting terminal 27 outside. In this example, the respective sliding surfaces of the second ring 41 are both smooth

The bottom of this second air gap 42, at the second connecting section 5, is equipped with a pair of second discharging openings 48 arranged symmetrically, to prevent an air bag from forming, able to lock the feeding of the second ring 41.

It is to be noted that at first the second ring 41 is fitted on the second tubular terminal 27 at the edge thereof 28.

The second ring 41, in turn, has a predetermined thickness, to adapt to the position of the second toroidal tooth 62, and a not circular contour, but which has at regular intervals fourth plane connecting surfaces 61, aimed at coupling in a prismatic relation with said third connecting surfaces 54 thereto they are perfectly complementary, this prevents the second ring 41 and the second breakable barrier 40 generally from rotating with the second tubular casing 27 as pin.

The second ring 41 is fitted with a certain interference on the seat thereof, to avoid a detachment of the second barrier 20.

On the outer surface of second tubular terminal 27, which is smooth, a second toroidal tooth 62 is further formed, near but however at a certain distance from the rim of the second edge 28, to avoid that the second ring 41 goes back and therefore that the barrier 21 breaks, in an assembling phase or due to an unintentional action, not determined by the operation of the device 1.

The second breakable barrier 40 further comprises a second membrane 43 extending over the whole area defined by said second ring 41, so as to wholly obstruct the second connecting opening 29.

A second rupture line 44, diametral to the second membrane 44, is implemented on the second membrane 43, determining the opening of the second barrier 40, and a pair of second peripheral rupture lines 45, arranged on the circular periphery of the second membrane 43, to ease the detachment between this one and the second ring 41.

Again, these two types of rupture lines are aimed at determining a localized separation between the second membrane 43 and the second ring 41 and between two portions of the same second membrane 43.

In order to keep such portions of the second membrane 43, which will open like a petal, joined to the ring, second anchoring points 50 are provided, determined by the interruptions of said second peripheral rupture lines 45.

The separation of said membrane portions, hereinafter first petal-like strips 58, starts at second points 59 for triggering the separation, which in the present example are determined by the intersections between the second cross rupture line 44 and the second peripheral rupture lines 45.

It is to be noted that the position of said second triggering points 59 will correspond exactly to that of the points of the second sharp profiles 52 on the second edge 28 of the second tubular casing 27. For this reason, that is to keep this correspondence, the ring 41 has to be prevented from rotating. Therefore, the cooperation between the first and the second connecting surfaces 54, 61 allows performing automatically the assembly of the second breakable barrier 40 in a correct position.

The rupture mechanism of the second breakable barrier 40 is the following one: when the second ring 41 is pushed into sliding towards the second connecting section 5 from the axial sliding between the connecting devices 2, 4, the second rupture lines 44 and 45, due to the effect of the incisions caused by the second sharp profiles 52 on the second triggering points 59 of the second membrane 43, separate, but the second membrane 43, separated in two petal-like strips 58, is dragged too by the second ring 41 leaving uncovered the second opening 29 which before was sealed.

The arrangement of the terminals and of the tubular casings 12, 17, 27, 32 of the first and of the second connecting member 2, 4 is so as to implement the rupture in sequence of the first barrier 20 and, subsequently, of the second one.

In this sequence, the first components which come in contact (figure 2A) are the ring 21 and the fourth ending edge 33 of the second tubular casing 32: this interaction causes the opening of the first membrane 24 which, with the continuation of the axial sliding, at first opens by spreading the four strips thereof 56, eve due to the effect of the interaction between first membrane 24 and first sharp profiles 51 on the respective first triggering points 57, and then it remains trapped in a position folded by 90° with respect to the starting position between the surfaces of the first and of the second tubular casing 17, 32.

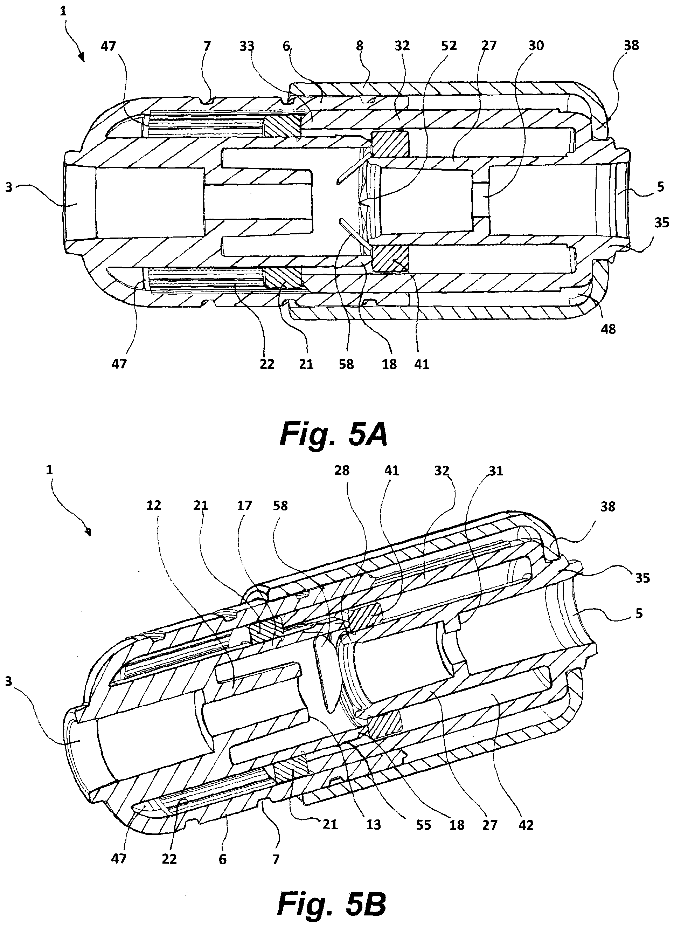

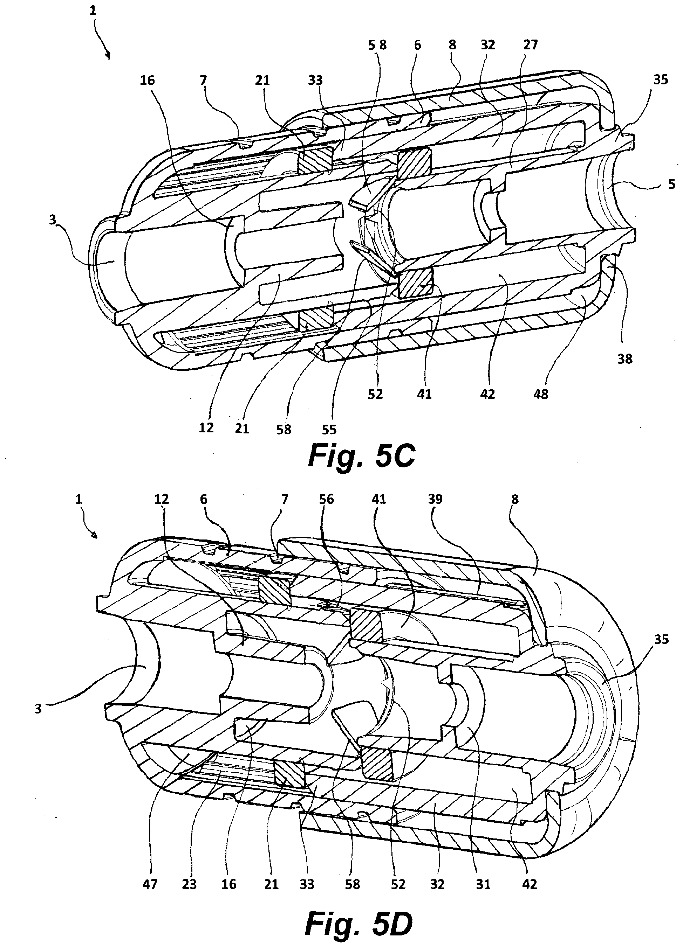

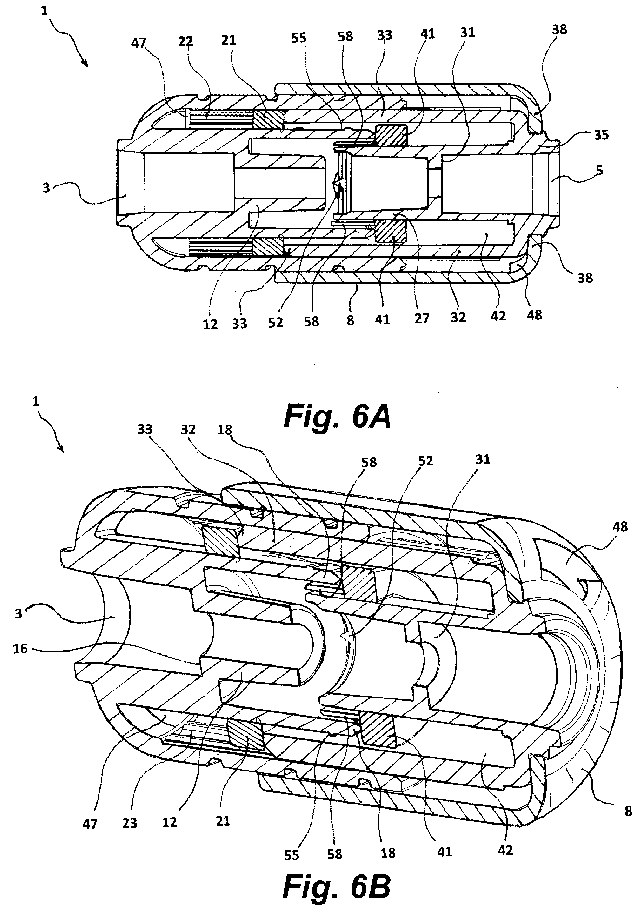

Upon continuing with this sequence, the third circular ending edges 18 come in contact with the second ring 41 by pushing it: the membrane 42 thus separates into two portions 58 (figure 5A) which open and then, tilted by 90°, remain trapped between the faced surfaces of the first and of the second tubular terminal 17, 27 (figure 6B).

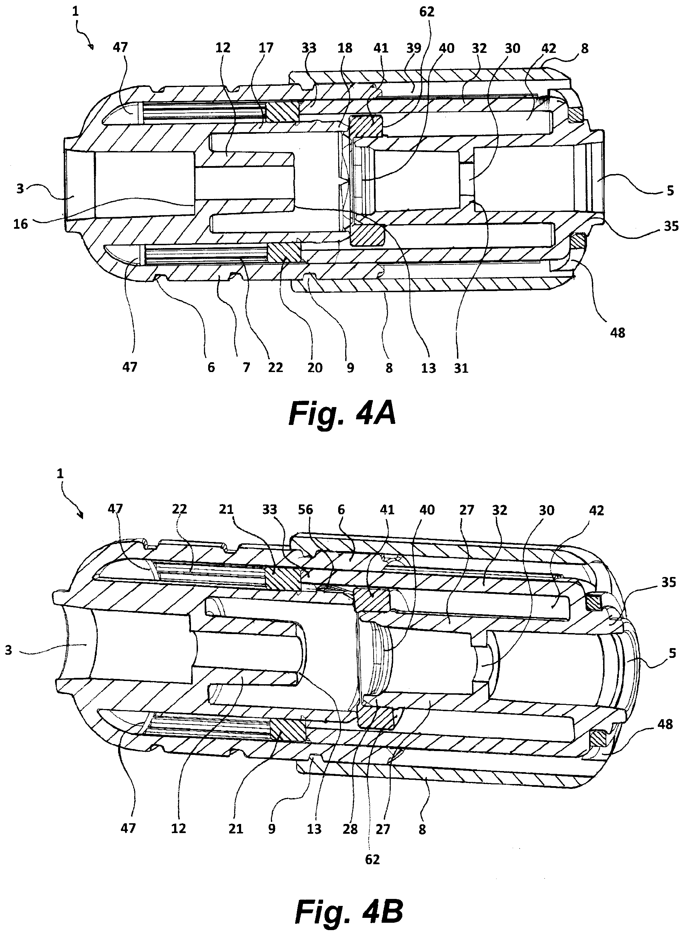

By comparing the two connecting members 2, 4 it is noted that the second tubular terminal 27 has a diameter smaller than the one of said first tubular casing 17 and larger than that of the first tubular terminal 12. Therefore, due to the effect of the axial sliding between the connecting devices 2, 4 induced by the screw coupling between the outer casings 6, 8, the second ending edge 28 of the second tubular terminal 27 is apt to approach to the third connecting opening 19 in the position occupied before by the first barrier 20 as far as penetrating therethrough (figure 4A); subsequently, the second edge 28 of the first tubular casing 27 penetrates through said third opening 19 (figure 5A, 5B and 5C) and the second sharp profiles 52 cause the rupture of the second breakable membrane 43, as detailed previously, which starts to slide inside said second air gap 42: the relation between the outer and inner diameters of terminals and casings being so as to keep a substantial tight inside said first tubular casing 17 during this sliding.

Still subsequently, the second terminal 27 continues its stroke inside the first tubular casing 17 as far as encountering the first connecting opening 14 and the circular edge thereof 13.

To this purpose, the first tubular terminal 12 is moved back with respect to the third circular ending edge 18 of the first tubular casing 17, and it has a smaller diameter than that of said second tubular terminal 27.

Therefore, the first tubular terminal 12 is able to penetrate (figures 6A and 6B) through the second opening 29 thanks to the previous rupture of the respective second breakable membrane 43 due to the effect of the motion of the second ring 41 as explained previously; at the same time the second ring 41 can slide in the second air gap 42 by dragging therewith the second membrane 43 by now separated into two strips 58: the relation between the outer and inner diameters of the two tubular terminals 12, 27 being so as to keep a substantial tight inside said first tubular terminal 12 during this sliding.

In this way, upon the rupture of the second breakable membrane 43, the inner connection to the connecting device 1 is created without the inside of the respective connecting members 2, 4 and of the related (not represented) ducts is opened to the outer environment.

A possible disconnection is implemented by rotating the second outer casing 8 in the opposite direction so as to disengage the above-described screw coupling.

The components of the above-described device could be easily produced by hot moulding, for example by using a polycarbonate or equivalent material. The breakable barriers could be produced instead with a high-density polyethylene (HDPE) or a polyethylene terephthalate (PTFE).

To the above-described connecting device a person skilled in the art, in order to satisfy additional and contingent needs, could bring several modifications and variants, all however comprised in the protection field of the present invention, as defined by the enclosed claims.

## Claims

1. A connecting device (1) for connecting therebetween the respective ends of two ducts comprising:
• a first and a second connecting member (2, 4), apt to cooperate mechanically therebetween in axial sliding, each one comprising a respective first and second section (3, 5) for the connection to first and second ends of two ducts;
• a first and a second tubular terminal (12, 27), formed in the respective first and second connecting member (2, 4), hydraulically connected to said first and second connecting member (3, 5), each one equipped with a corresponding first and second ending circular edge (13, 28) with pre-fixed diameter defining respective first and second connecting openings (14, 29), the second connecting opening (29) being sealed by a breakable barrier (40) connected to the second ending circular edge (28); and
• a first tubular casing (17) concentric to said first tubular terminal (12), equipped with a third ending circular edge (18) defining a third connecting opening (19) sealed by an additional breakable barrier (20) connected to the third ending circular edge (18),
wherein the second tubular terminal (27) has a smaller diameter than that of said first tubular casing (17), being apt to penetrate through said third connecting opening (18) consequently to said axial sliding, causing the rupture of said additional breakable barrier (20) and sliding sealingly inside said first tubular casing (17); and
wherein the first tubular terminal (12) is moved back with respect to the third ending circular edge (18) of the first tubular casing (17), having a smaller diameter than that of said second tubular terminal (27) and being apt to penetrate through said second connecting opening (29) consequently to said axial sliding, causing the rupture of the respective breakable barrier (40) and sliding sealingly inside said second tubular terminal (27) and,
wherein at least one of said first and second connecting member (2, 4) comprises a sliding guide,
**characterized in that** said sliding guide comprises at least a groove formed on a surface of an outer cylindrical casing (6) of a connecting member, apt to cooperate with a corresponding rib or with one or more teeth (9) implemented at an inner surface of an outer cylindrical casing (8) of the other connecting member (4).

2. The connection device (1) according to claim 1, wherein said sliding guide is helical, thus producing a screw coupling between the connecting members.

3. The connection device (1) according to claim 1, wherein said third opening (19) is positioned inside the first connecting member (2), in an intermediate position between an inserting mouth thereof (10), with respect thereto it is moved back, and said first connecting opening (14).

4. The connection device (1) according to claim 1, wherein said breakable barriers (20, 40) comprise a ring (21, 41) arranged to slide in a respective air gap (22, 42) outwardly said first casing and/or second tubular terminal (17, 27), the air gap having discharging openings (47, 48), to prevent an air pocket from forming.

5. The connection device (1) according to claim 4, wherein the rings (21, 41) are arranged to slide in a relation of prismatic pair in said connecting members (2, 4).

6. The connection device (1) according to claim 4, wherein said breakable barriers (20, 40) comprise a respective membrane (24, 43) extending on the whole area defined by the respective ring (21, 41), whereon cross rupture lines (25, 44) are implemented, as far as the membrane (24, 43) and peripheral rupture lines (26 45), arranged on the circular perimeter of the membrane (24, 43), to ease the detachment of this one from the ring (21, 41).

7. The connection device (1) according to claim 6, wherein the peripheral rupture lines (26 45) do not surround the whole perimeter of the respective breakable membrane (24, 43) and they leave free junction points (49,50) so that the connection between ring (21, 41) and breakable membrane (24, 43) does not interrupt, so that the breakable membrane (24, 43) can bend by 90° and, after having been dragged by the respective ring (21, 41), remains trapped between the respective tubular terminals (12, 27) or casings (17, 32) of the first and second connecting member (2, 4).

8. The connection device (1) according to claims 5 and 6, wherein rupture-triggering points (57, 59) are formed on said membranes (24, 43), which points correspond to respective sharp profiles (51, 52) formed at the edges (18, 28) whereon said breakable barriers (20, 40) are arranged.

9. The connection device (1) according to claim 1 or 2, wherein the connecting members (2, 4) have casings (6, 32) apt to slide in contact one with respect to the other one, having on the respective surfaces in contact rectilinear ribs and grooves (23, 39) cooperating therebetween to form a prismatic pair.

10. The connection device (1) according to claim 2, wherein a connecting member (2, 4) has an outer casing (8) free to rotate to implement said screw coupling, whereas the connecting member (4) slides axially.

11. The connection device (1) according to claim 5, wherein the rings (21, 41) of the breakable barriers (20, 40) are kept in their position, before the sliding thereof, by a respective first toroidal tooth (55, 62).

## Patentansprüche

1. Verbindungsvorrichtung (1) zum Befestigen von jeweiligen Enden von zwei Kanälen dazwischen umfassend:
ein erstes und ein zweites Verbindungselement (2, 4), die geeignet sind dazwischen mechanisch bei axialem Gleiten zusammenzuarbeiten, wobei jedes einen jeweiligen erst in und zweiten Bereich (3, 5) für die Verbindung zu ersten und zweiten Enden der zwei Kanäle umfasst;
ein erstes und ein zweites rohrförmigen Ende (12, 27), die in den jeweiligen ersten und zweiten Verbindungselementen (2, 4) gebildet sind, hydraulisch mit dem ersten und zweiten Verbindungselement (3, 5) verbunden, wobei jedes mit einer korrespondierenden ersten und zweiten runden Endkante (13, 28) mit festgelegten Durchmesser ausgestattet ist, die jeweilige erste und zweite Verbindungsöffnungen (14, 29) definieren, wobei die zweite Verbindungsöffnung (29) durch eine brechbare Barriere (40) verschlossen ist, die mit der zweiten runden Endkante (28) verbunden ist; und
ein erstes rohrförmiges Gehäuse (17), das zu dem ersten rohrförmigen Ende (12) konzentrisch ist und mit einer dritten runden Endkante (18) ausgestattet ist, die eine dritte Verbindungsöffnung (19) definiert, die durch eine zusätzliche brechbare Barriere (20), die mit der dritten runden Endkante (18) verbunden ist, verschlossen ist,
wobei das zweite rohrförmige Ende (27) einen kleineren Durchmesser aufweist, als den des ersten rohrförmigen Gehäuses (17), dazu geeignet, durch die dritte Verbindungsöffnung (18) folgend auf das axiale Gleiten hindurch zu dringen, wobei das Brechen der zusätzlichen brechbaren Barriere (20) und ein abgedichtetes Gleiten innerhalb des ersten rohrförmigen Gehäuses (17) verursacht wird; und
wobei das erste rohrförmige Ende (12) bezüglich der dritten runden Endkante (18) des ersten rohrförmigen Gehäuses (17), das einen kleineren Durchmesser als den des zweiten rohrförmigen Endes (27) aufweist, zurück versetzt ist, und geeignet ist durch die zweite Verbindungsöffnung (29) folgend auf das axiale Gleiten hindurch zu treten, wobei das Brechen der jeweiligen brechbaren Barriere (40) und abgedichtetes Gleiten innerhalb des zweiten rohrförmigen Endes (27) verursacht wird und,
wobei zumindest eines des ersten und zweiten Verbindungselementes (2, 4) eine Gleitführung umfasst,
**dadurch gekennzeichnet dass** die Gleitführung zumindest eine Nut umfasst, die auf einer Oberfläche eines äußeren zylindrischen Gehäuses (6) eines Verbindungselementes gebildet ist, geeignet um mit einer korrespondierenden Rippe oder mit einem oder mehreren Zähnen (9), die an einer inneren Oberfläche eines äußeren zylindrischen Gehäuses (8) des anderen Verbindungelementes (4) implementiert sind, zusammenzuarbeiten.

2. Verbindungsvorrichtung (1) gemäß Anspruch 1, wobei die Gleitführung spiralförmig ist und somit eine Schraubverbindung zwischen den Verbindungselementen erzeugt.

3. Verbindungsvorrichtung (1) gemäß Anspruch 1, wobei die dritte Öffnung (19) innerhalb des ersten Verbindungselementes (2) in einer Zwischenposition zwischen einem Einführungsmund davon (10), bezüglich dessen diese zurück versetzt ist, und der zweiten Verbindungsöffnung (14) positioniert ist.

4. Verbindungsvorrichtung (1) gemäß Anspruch 1, wobei die brechbaren Barrieren (20, 40) einen Ring (21, 41) umfassen, der angeordnet ist, um in einem jeweiligen Luftspalt (22, 42) nach außen von dem ersten Gehäuse und/oder zweiten rohrförmigen Ende (17, 27) zu gleiten, wobei der Luftspalt Auslassöffnungen (47, 48) aufweist, um das Bilden einer Lufttasche zu verhindern.

5. Verbindungsvorrichtung (1) gemäß Anspruch 4, wobei die Ringe (21, 41) angeordnet sind, um in einer Relation eines prismatischen Paares in den Verbindungselementen (2, 4) zu gleiten.

6. Verbindungsvorrichtung (1) gemäß Anspruch 4, wobei die brechbaren Barrieren (20, 40) eine jeweilige Membranen (24, 43) umfassen, die sich an der gesamten Fläche erstrecken, die durch den jeweiligen Ring (21, 41) definiert ist, wobei Kreuzreißlinien (25, 44) implementiert sind bis zu der Membran (24, 43) und peripheren Reißlinien (26, 45), die auf dem runden Umfang der Membran (24, 43) angeordnet sind, um das Lösen hiervon von dem Ring (21, 41) zu erleichtern.

7. Verbindungsvorrichtung (1) gemäß Anspruch 6, wobei die peripheren Reißlinien (26, 45) nicht den gesamten Umfang der jeweiligen brechbaren Membran (24, 43) umgibt und diese Übergangspunkte (49, 50) freilässt, so dass die Verbindung zwischen Ring (21, 41) und brechbarer Membran (24, 43) nicht unterbrochen ist, so dass die brechbare Membran (24, 43) um 90° gebogen werden kann und, nachdem diese durch den jeweiligen Ring (21, 41) gezogen wurde, zwischen den jeweiligen rohrförmigen Enden (12, 27) oder Gehäusen (17, 32) des ersten und zweiten Verbindungselementes (2, 4) eingefangen bleibt.

8. Verbindungsvorrichtung (1) gemäß Anspruch 5 und 6, wobei Riss-Triggerpunkte (57, 59) auf den Membranen (24, 43) gebildet sind, wobei die Punkte mit entsprechenden scharfen Profilen (51, 52) korrespondieren, die an den Kanten (18, 28) gebildet sind, auf denen die berechenbaren Barrieren (20, 40) angeordnet sind.

9. Verbindungsvorrichtung (1) gemäß Anspruch 1 oder 2, wobei die Verbindungselemente (2, 4) Gehäuse (6, 32) aufweisen, die geeignet sind, dass eines bezüglich des anderen in Kontakt gleitet, wobei diese auf den jeweiligen Oberflächen, die in Kontakt stehen, gradlinige Rippen und Nuten (23, 39) aufweisen, die miteinander zusammenwirken, um ein prismatisches Paar zu bilden.

10. Verbindungsvorrichtung (1) gemäß Anspruch 2, wobei ein Verbindungselement (2, 4) ein äußeres Gehäuse (8) aufweist, das frei ist, um sich zu drehen, um die Schraubverbindung zu implementieren, während das Verbindungselement (4) axial gleitet.

11. Verbindungsvorrichtung (1) gemäß Anspruch 5, wobei die Ringe (21, 41) der berechenbaren Barrieren (20, 40) durch einen entsprechenden ersten ringförmigen Zahn (55, 62) in deren Position gehalten werden, bevor diese gleiten.

## Revendications

1. Dispositif de raccordement (1) pour raccorder entre elles les extrémités respectives de deux conduits comprenant :
- un premier et un deuxième élément de raccordement (2, 4), aptes à coopérer mécaniquement entre eux en un coulissement axial, chacun comprenant des première et deuxième sections (3, 5) respectives pour le raccordement aux première et deuxième extrémités de deux conduits ;
- un premier et un deuxième embout tubulaire (12, 27), formés dans les premier et deuxième éléments de raccordement (2, 4) respectifs, hydrauliquement raccordés auxdits premier et deuxième éléments de raccordement (3, 5), chacun étant équipé de premier et deuxième bords circulaires de terminaison (13, 28) correspondants avec un diamètre préfixé définissant des première et deuxième ouvertures de raccordement (14, 29) respectives, la deuxième ouverture de raccordement (29) étant scellée par une barrière cassable (40) raccordée au deuxième bord circulaire de terminaison (28); et
- un premier boîtier tubulaire (17) concentrique avec ledit premier embout tubulaire (12), équipé d'un troisième bord circulaire de terminaison (18) définissant une troisième ouverture de raccordement (19) scellée par une barrière cassable supplémentaire (20) raccordée au troisième bord circulaire de terminaison (18),
dans lequel le deuxième embout tubulaire (27) a un diamètre plus petit que celui dudit premier boîtier tubulaire (17), étant apte à pénétrer à travers ladite troisième ouverture de raccordement (18) à la suite dudit coulissement axial, entraînant la rupture de ladite barrière cassable supplémentaire (20) et coulissant de manière étanche à l'intérieur dudit premier boîtier tubulaire (17) ; et
dans lequel le premier embout tubulaire (12) est reculé par rapport au troisième bord circulaire de terminaison (18) du premier boîtier tubulaire (17), ayant un diamètre plus petit que celui dudit deuxième embout tubulaire (27) et étant apte à pénétrer à travers ladite deuxième ouverture de raccordement (29) à la suite dudit coulissement axial, entraînant la rupture de la barrière cassable (40) respectives et coulissant de manière étanche à l'intérieur dudit deuxième embout tubulaire (27) et,
dans lequel au moins un desdits premier et deuxième éléments de raccordement (2, 4) comprend un guide de coulissement,
**caractérisé en ce que** ledit guide de coulissement comprend au moins une rainure formée sur une surface d'un boîtier cylindrique extérieur (6) d'un élément de raccordement, apte à coopérer avec une nervure correspondante ou avec une ou plusieurs dents (9) implémentées au niveau d'une surface intérieure d'un boîtier cylindrique extérieur (8) de l'autre élément de raccordement (4).

2. Dispositif de raccordement (1) selon la revendication 1, dans lequel ledit guide de coulissement est hélicoïdal, produisant ainsi un accouplement à vis entre les éléments de raccordement.

3. Dispositif de raccordement (1) selon la revendication 1, dans lequel ladite troisième ouverture (19) est positionnée à l'intérieur du premier élément de raccordement (2), dans une position intermédiaire entre une embouchure d'insertion de celui-ci (10), par rapport à laquelle il est reculé, et ladite première ouverture de raccordement (14).

4. Dispositif de raccordement (1) selon la revendication 1, dans lequel lesdites barrières cassables (20, 40) comprennent une bague (21, 41) agencée pour coulisser dans un entrefer (22, 42) respectif vers l'extérieur dudit premier boîtier et/ou dudit deuxième embout tubulaire (17, 27), l'entrefer ayant des ouvertures de décharge (47, 48), pour empêcher la formation d'une poche d'air.

5. Dispositif de raccordement (1) selon la revendication 4, dans lequel les bagues (21, 41) sont agencées pour coulisser dans une relation de paire prismatique dans lesdits éléments de raccordement (2, 4).

6. Dispositif de raccordement (1) selon la revendication 4, dans lequel lesdites barrières cassables (20, 40) comprennent une membrane (24, 43) respective s'étendant sur toute la superficie définie par la bague (21, 41) respective, sur laquelle des lignes de rupture en croix (25, 44) sont implémentées, jusqu'à la membrane (24, 43) et des lignes de rupture périphériques (26, 45), agencées sur le périmètre circulaire de la membrane (24, 43), pour faciliter la séparation de celle-ci de la bague (21,41).

7. Dispositif de raccordement (1) selon la revendication 6, dans lequel les lignes de rupture périphériques (26, 45) n'entourent pas tout le périmètre de la membrane cassable (24, 43) respective et elles laissent des points de jonction (49, 50) libres de telle sorte que le raccordement entre la bague (21, 41) et la membrane cassable (24, 43) ne s'interrompt pas, de telle sorte que la membrane cassable (24, 43) peut plier de 90° et, après avoir été traînée par la bague (21, 41) respective, reste coincée entre les embouts tubulaires (12, 27) ou boîtiers (17, 32) respectifs des premier et deuxième éléments de raccordement (2, 4).

8. Dispositif de raccordement (1) selon les revendications 5 et 6, dans lequel des points de déclenchement de rupture (57, 59) sont formés sur lesdites membranes (24, 43), lesquels points correspondent à des profils tranchants (51, 52) respectifs formés au niveau des bords (18, 28) sur lesquels lesdites barrières cassables (20, 40) sont agencés.

9. Dispositif de raccordement (1) selon la revendication 1 ou 2, dans lequel les éléments de raccordement (2, 4) ont des boîtiers (6, 32) aptes à coulisser en contact l'un par rapport à l'autre, ayant sur les surfaces respectives en contact des nervures et rainures rectilignes (23, 39) coopérant entre elles pour former une paire prismatique.

10. Dispositif de raccordement (1) selon la revendication 2, dans lequel un élément de raccordement (2, 4) comporte un boîtier extérieur (8) libre de tourner pour implémenter ledit accouplement à vis, alors que l'élément de raccordement (4) coulisse axialement.

11. Dispositif de raccordement (1) selon la revendication 5, dans lequel les bagues (21, 41) des barrières cassables (20, 40) sont maintenues dans leur position, avant leur coulissement, par une première dent toroïdale (55, 62) respective.
